(19) Europäisches Patentamt
European Patent Office
Office européen des brevets

(11) EP 0 787 493 A1

(12) EUROPEAN PATENT APPLICATION

(43) Date of publication:
06.08.1997 Bulletin 1997/32

(21) Application number: 97100926.1

(22) Date of filing: 22.01.1997

(51) Int. Cl.[6]: **A61K 31/47**, C07D 221/12, C07D 221/16, C07D 217/20, C07D 217/18, C07D 217/04, C07D 491/04

(84) Designated Contracting States:
AT BE CH DE DK ES FI FR GB GR IE IT LI LU MC NL PT SE

(30) Priority: 03.02.1996 EP 96101553

(71) Applicant: F. HOFFMANN-LA ROCHE AG
4070 Basel (CH)

(72) Inventors:
- Alanine, Alexander
68400 Riedisheim (FR)
- Bourson, Anne
68100 Mulhouse (FR)
- Büttelmann, Bernd
79650 Schopfheim (DE)
- Fischer, Günther
79541 Lörrach-Brombach (DE)
- Heitz Neidhart, Marie-Paule
68220 Hagenthal 1e Bas (FR)
- Mutel, Vincent
68100 Mulhouse (FR)
- Pinard, Emmanuel
68300 Saint-Louis (FR)
- Röver, Stephan
79594 Inzlingen (DE)
- Trube, Gerhard
79618 Rheinfelden (DE)
- Wyler, René
8002 Zürich (CH)

(74) Representative: Poppe, Regina et al
F.Hoffmann-La Roche AG
Patent Department(PLP),
124 Grenzacherstrasse
4070 Basel (CH)

# (54) Tetrahydroisoquinoline derivatives

(57) The present invention relates to the use of tetrahydroisoquinoline derivatives of the general formula

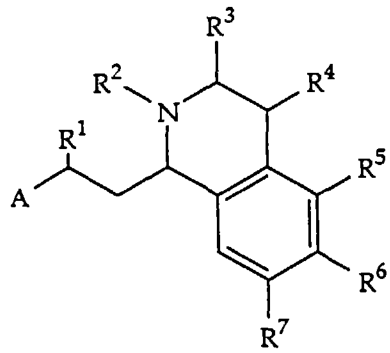

I

wherein

| | |
|---|---|
| A | is aryl |
| $R^1$ | is hydrogen, hydroxy, lower alkyl, lower alkoxy, R-CO- or R-COO-, wherein R is lower alkyl; |
| $R^2$ | is hydrogen, lower alkyl or cycloalkyl |
| $R^3$-$R^7$ | are hydrogen, lower alkyl, lower alkoxy, hydroxy or |
| $R^3$ and $R^4$ | taken together are $-(CH_2)_n-$ or |
| $R^6$ and $R^7$ | taken together are $-OCH_2O-$ and |
| n | is 3 or 4, |

EP 0 787 493 A1

as well as pharmaceutically acceptable salts for the manufacture of medicaments for the control or treatment of diseases which represent therapeutic indications for NMDA receptor subtype specific blockers.

## Description

The invention relates to compounds of the general formula

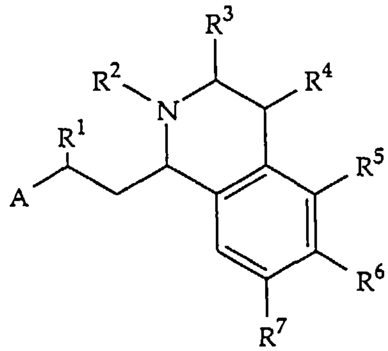

I

wherein

| | |
|---|---|
| A | is aryl |
| $R^1$ | is hydrogen, hydroxy, lower alkyl, lower alkoxy, R-CO- or R-COO-, wherein R is lower alkyl; |
| $R^2$ | is hydrogen, lower alkyl or cycloalkyl |
| $R^3$-$R^7$ | are hydrogen, lower alkyl, lower alkoxy, hydroxy or |
| $R^3$ and $R^4$ | taken together are $-(CH_2)_n-$ or |
| $R^6$ and $R^7$ | taken together are $-OCH_2O-$ and |
| n | is 3 or 4, |

or pharmaceutically acceptable salts thereof.

Most of the above described isoquinoline derivatives and their salts are known compounds. In US patents 3.238.212, 3.067.203 and 3.217.007 these compounds are stated to possess analgesic, spasmolytic and antitussive activities. Mol. Pharmacol. (1976), 12(5), 854-61 describes tests of tetrahydroisoquinolines for agonist and antagonist activity with dopamine and beta adenylate cyclase system.

It has now surprisingly been found that compounds of the present invention are NMDA-R subtype selective blockers.

NMDA receptors have a key function in modulating neuronal activity and plasticity which makes them key players in mediating processes underlying development of CNS as well as learning and memory formation. Under pathological conditions of acute and chronic forms of neurodegeneration overactivation of NMDA receptors is a key event for triggering neuronal cell death.

NMDA receptors are composed of members from two subunit families, namely NR-1 (8 different splice variants) and NR-2 (A to D) originating from different genes. Members from the two subunit families show a distinct distribution in different brain areas. Heteromeric combinations of NR-1 members with different NR-2 subunits result in NMDA receptors, displaying different pharmacological properties.

Possible therapeutic indications for NMDA receptor subtype specific blockers include acute forms of neurodegeneration caused e.g. by stroke and brain trauma, and chronic forms of neurodegeneration such as Alzheimer's disease, Parkinson's disease, Huntington's disease, ALS (amyotrophic laterial slcerosis) and neurodegeneration associated with bacterial or viral infections, and, in addition, therapeutic indications such as schizophrenia, anxiety and depression.

Compounds of the present invention are therefore useful in the treatment of acute forms of neurodegeneration caused e.g. by stroke and brain trauma, and chronic forms of neurodegeneration such as Alzheimer's disease, Parkinson's disease, Huntington's disease, ALS (amyotrophic laterial slcerosis) and neurodegeneration associated with bacterial or viral infections, and, in addition, therapeutic indications such as schizophrenia, anxiety and depression.

Objects of the present invention are the use of compounds of formula I in the treatment or prophylaxis of diseases caused by overactivation of respective NMDA receptor subtypes, such as acute forms of neurodegeneration caused e.g. by stroke and brain trauma, and chronic forms of neurodegeneration such as Alzheimer's disease, Parkinson's disease, Huntington's disease, ALS (amyotrophic laterial slcerosis) and neurodegeneration associated with bacterial or viral infections, and, in addition, therapeutic indications such as schizophrenia, anxiety and depression the use of these compounds for manufacture of corresponding medicaments, and medicaments containing these compounds. Objects of the present invention are also the new compounds of formula

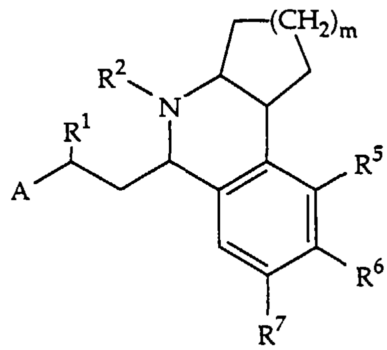

Ia

wherein A, $R^1$, $R^2$ and $R^5$-$R^7$ are described as above and m is 1 or 2.

In another aspect, the present invention relates to a method of reducing acute or chronic forms of neurodegeneration which comprises administering to a host in need of such treatment an effective amount of a compound of formula I.

The following definitions of the general terms used in the present description apply irrespective of whether the terms in question appear alone or in combination.

As used herein, the term "lower alkyl" denotes a straight or branched-chain alkyl group containing from 1 to 4 carbon atoms, for example, methyl, ethyl, propyl, isopropyl, butyl and the like. The term "aryl" denotes an aromatic hydrocarbon residue, preferably phenyl, which may be unsubstituted or substituted by one or more (up to three) substitutents, selected from hydroxy lower alkyl, halogen, lower alkoxy or nitro.

The term "halogen" denotes chlorine, iodine, fluorine or bromine. The term "lower alkoxy" denotes an alkyl group, as defined earlier wich is attached via an oxygen atom. The term "cycloalkyl" denotes saturated cyclic hydrocarbon residues containing 3 to 6 carbon atoms.

The tetrahydroisoquinoline compounds of formula I contain two asymmetric carbon atoms. Accordingly, the formation of two stereoisomeric racemates is possible. The present invention embraces all possible racemates and their optical antipodes.

Exemplary preferred compounds are:

2-(6,7-Dimethoxy-2-methyl-1,2,3,4-tetrahydroisoquinolin-1-yl)-1-p-tolyl-ethanol;
1-[2-(4-Chloro-phenyl)-ethyl]-6-methoxy-2-methyl-1,2,3,4-tetrahydroisoquinolin-7-ol;
1-(4-Chloro-phenyl)-2-(6,7-dimethoxy-2-methyl-1,2,3,4-tetrahydroisoquinolin-1-yl)-ethanol;
1-[2-(4-Chloro-phenyl)-ethyl] -2-methyl-1,2,3,4-tetrahydro-isoquinoline-6,7-diol;
6,7-Dimethoxy-2-methyl-1-(2-p-tolyl-ethyl)-1,2,3,4-tetrahydroisoquinoline;
6-[2-(4-Chloro-phenyl)-ethyl]-8,9-dimethoxy-5-methyl-1,2,3,4,4a,5,6,10b-octahydro-phenanthridine;
2-(6,7-Dimethoxy-2-methyl-1,2,3,4-tetrahydroisoquinolin-1-yl)-1-(4-nitrophenyl)-ethanol; and
6-[2-(4-Chloro-phenyl)-ethyl]-8,9,dimethoxy-1,2,3,4,4a,5,6,10b-octahydrophenanthridine.

The present compounds of formula I and their pharmaceutically acceptable salts can be prepared by processes, described in the above mentioned references, for example, in US 3,238,212 or 3,217,007 is described a process which comprises reacting a dihydroisoquinolinium compound of the general formula

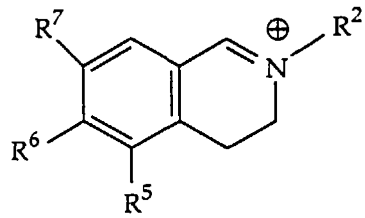

II

wherein $R^2$ and $R^5$-$R^7$ are as described above,
with a ketone having the formula $CH_3COA$, wherein A is as described above, in the presence of a basic condensation agent.

The reduction of the oxo group to a hydroxy group is, in the practice of the present invention, carried out by methods which are known per se. It is expedient, however, to accomplish the reduction of the starting material using an alkali-metal-metal hydride, such as lithium aluminum hydride or, especially, sodium borohydride, potassium borohydride etc.

A preferred method comprises carrying out the reduction using sodium borohydride in the presence of a solvent which is stable in the presence of the reducing agent. Suitable solvents include, for example, methanol, ethanol or dimethylformamide. After the reduction has been carried out any aralkyloxy group, especially the benzyloxy group, can be cleaved readily by hydrogenolysis to provide free hydroxy groups.

Such debenzylation is carried out advantageously, catalytically, for example, in the presence of a noble metal catalyst, such as palladium. In an additional procedural step, the compound can be esterified. The esters can be prepared by reacting with a conventional acylating agent.

Compounds of formula I and their pharmaceutically acceptable salts can also be prepared by a process which comprises cyclizing the acid amide of formula

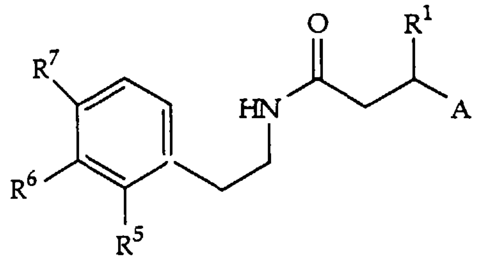

III

wherein A, $R^1$ and $R^5$-$R^7$ are as described above,

in the presence of an acid, preferably $POCl_3$, to the corresponding 1-phenyl-ethyl-3,4-dihydroisoquinoline derivative and the latter compound is subsequently reduced with a suitable reducing agent, such as an alkali metal-metal hydride, for example, sodium borohydride.

The novel compounds of formula IA can be prepared by a process which comprises cyclizing the acid amide of formula

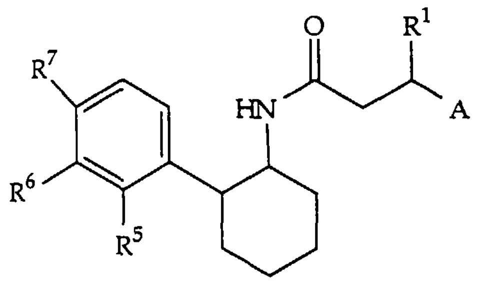

IV

in the presence of an acid, preferably $POCl_3$, as described above for cyclizing the acid amide of formula III.

As described above, the tetrahydroisoquinolines of formula I contain two asymmetric carbon atoms and the formation of two stereoisomeric racemates is possible. If these racemates form concurrently, they can be separated by methods known per se, for example, by chromatography or by fractional crystallization. The racemates themselves, can, if desired, be separated into their optical antipodes by methods known per se, for example, by fractional crystallization of the salts with optically active acids, such as α-tartaric acid, dibenzoyl-α-tartaric acid or α-camphorsulfonic acid.

The compounds of formula I can be converted into pharmaceutically acceptable acid addition salts. These salts can be manufactured according to methods which are known per se and which will be familiar to any person skilled in the art.

The activity of compounds of formula I can be demonstrated by the following:

3H-MK801 (Dizocilpine) binding in vitro

The whole brain from 150-200g male rats, without cerebellum and without medulla oblongata was dissected on ice. The tissue was then homogenized with an Ultra-Turrax maximum speed during 30 seconds at 4°C in 50 volumes of cold Tris HCl 50mM, EDTA disodium 10mM, pH=7.4 buffer (wet weight/v). The homogenate was centrifuged at 48'000 x g (20'000 rpm, SS34, Sorvall RC5C) for 10 minutes. The pellet was rehomogenized with the same volume of buffer and the homogenate incubated at 37°C for 10 minutes. After centrifugation as above, the pellet was rehomogenized with the same volume of buffer and frozen at -80°C in 35 ml fractions for at least 16 hours and not more than 2 weeks.

For the binding experiment, the homogenate was centrifuged as above and the pellet was washed 3 times by homogenization in 25 volumes of cold Tris HCl 5mM, pH=7.4 buffer (Ultra-Turrax, maximum speed, 30 seconds) and

centrifugation as above. The final pellet was rehomogenized in 25 volumes of buffer (original wet weight) and used as such in the assay. The final concentration of membrane in the assay was 20mg/ml (wet weight).

The incubation was performed in the presence of 1nM glutamate, glycine and spermidine. MK-801, (+)-[3-3H(N)], NEN (NET-972) 20Ci/mmol, was used at 5nM final concentration. Non specific binding was determined in presence of 100mM TCP. After 2 hours of incubation at room temperature, the suspension was filtered (Whatmann GF/B, soaked in 0.1% polyethylenimine for 2 hours) and washed 5 times with 3 ml of cold Tris HCl 5mM, pH=7.4 buffer. The air-dried filters were counted with 10ml of Ultima-gold (Packard) in a Tri-Carb 2500 TR scintillation counter after agitation.

The DPM were transformed in % of specific binding and these values were treated by a non linear regression calculation program (BINDING, H. Affolter, Switzerland) which provided the $IC_{50}$ values for the low and high affinity binding sites (= concentrations producing half maximal inhibition at the respective sites). Each experiment was repeated at least three times and the final $IC_{50}$ values were calculated as the mean +/-standard deviation of the individual experiments.

Reference: R.W. Ransom and N.L. Stec. Journal of Neurochemistry 51, 830-836, 1988.

Electrophysiology on recombinant NMDA receptors.

cDNA clones coding for the subunits NMDAR1C and NMDAR2A of the NMDA receptor (see Hollmann and Heinemann, 1994, Annu. Rev. Neurosci. 17: 31 for nomenclature of NMDA receptor subunits) were isolated from a rat brain λgt11 cDNA library as published elsewhere (Sigel et al., 1994, J. Biol. Chem. 269:8204). The clone for the subunit NMDAR2B of the rat brain NMDA receptor was obtained from S.Nakanishi (Kyoto, Japan). The cDNAs were transcribed, capped and poly($A^+$)-tailed as described previously (Malherbe et al., 1990, Mol. Brain Res. 8: 199). Oocytes of South African frogs (Xenopus laevis) were used for expressing either a combination of the NMDAR1C and NMDAR2A subunits or the NMDAR1C and NMDAR2B subunits. Approximately 3 fmol of a 1:1 mixture of the respective mRNA species were injected into every oocyte. Four to five days later the ion current through the NMDA receptor channels was measured in voltage clamp experiments (see Methfessel et al., 1986, Pflügers Arch. 407: 577 for the methods of oocyte expression and voltage-clamping). The membrane potential was clamped to -80 mV and the receptors were activated by applying a modified Ringer's solution containing the agonists L-asparatate (Asp) and glycine (Gly). Different agonist concentrations were chosen for either subunit combination to account for the different agonist sensitivities of the two types of receptors (70 μM Asp plus 2.5 μM Gly for NMDAR1C - NMDAR2A and 15 μM Asp plus 0.2 μM Gly for NMDAR1C - NMDAR2B). The agonists were applied for 15 s intervals once every 2.5 min by rapid superfusion of the oocyte. After a series of initial control stimuli increasing concentrations of the antagonist to be tested were added to both, the basal Ringer's and the agonist containing solution. For the data analysis the amplitude (y) of the agonist-induced current was plotted versus the concentration (x) of the antagonist and the logistic function $y = A/[1 + (x/IC_{50})^H]$ was fitted to the data to estimate the 50 % inhibitory concentration ($IC_{50}$). Three to six oocytes were tested for every antagonist and if possible, at least 3 concentrations embracing the $IC_{50}$ were applied to every oocyte. However, concentrations higher than 100 μM were never used even if the $IC_{50}$ had not yet been reached at 100 μM and for two compounds the maximum concentration was even less (20-30 μM) because of limited solubility. In these cases a lower limit (e.g., ">100 μM") for the $IC_{50}$ is given in table "Test Results". In two other cases a concentration of 0.1 μM produced a slowly increasing block which exceeded 50% after 30 min. Because of the slow onset of block it was unreasonable to test even lower concentrations; instead an upper limit ("<0.1 μM") for the $IC_{50}$ is given in table "Test results". Figures for the $IC_{50}$ in all other cases are arithmetic mean values of individual $IC_{50}$s determined by the logistic curve fits.

Tested compounds of formula I

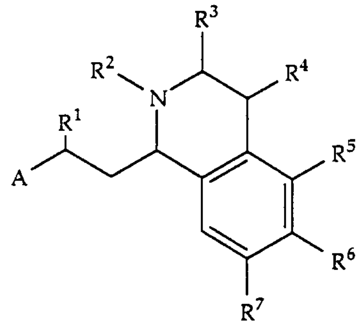

| Nr. | A | $R^1$ | $R^2$ | $R^3$ | $R^4$ | $R^5$ | $R^6$ | $R^7$ |
|---|---|---|---|---|---|---|---|---|
| A | 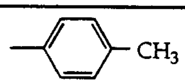 | OH | $CH_3$ | H | H | H | $OCH_3$ | $OCH_3$ |
| B | 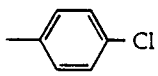 | H | $CH_3$ | H | H | H | $OCH_3$ | OH |
| C | 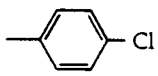 | OH | $CH_3$ | H | H | H | $OCH_3$ | $OCH_3$ |
| D | 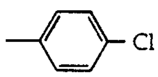 | H | $CH_3$ | H | H | H | OH | OH |
| E | 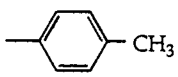 | H | $CH_3$ | H | H | H | $OCH_3$ | $OCH_3$ |
| F | 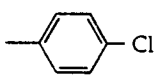 | H | $CH_3$ | together $-(CH_2)_4-$ | | H | $OCH_3$ | $OCH_3$ |
| G | 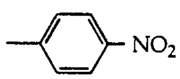 | OH | $CH_3$ | H | H | H | $OCH_3$ | $OCH_3$ |
| H | 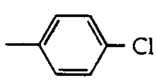 | H | H | together $-(CH_2)_4-$ | | H | $OCH_3$ | $OCH_3$ |
| I | 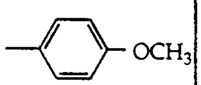 | OH | $CH_3$ | H | H | H | $OCH_3$ | $OCH_3$ |
| J | 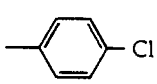 | H | $CH_3$ | H | H | H | $OCH_3$ | $OCH_3$ |

| Nr. | A | $R^1$ | $R^2$ | $R^3$ | $R^4$ | $R^5$ | $R^6$ | $R^7$ |
|---|---|---|---|---|---|---|---|---|
| K | 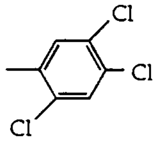 | OH | $CH_3$ | H | H | H | $OCH_3$ | $OCH_3$ |
| L | 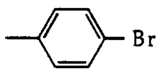 | H | $CH_3$ | H | H | H | $OCH_3$ | $OCH_3$ |
| M | 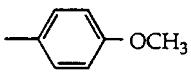 | H | $CH_3$ | H | H | H | $OCH_3$ | $OCH_3$ |
| N | 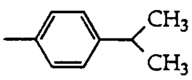 | H | $CH_3$ | H | H | H | $OCH_3$ | $OCH_3$ |
| O | 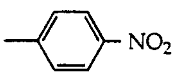 | H | $CH_3$ | H | H | H | $OCH_3$ | $OCH_3$ |
| P | 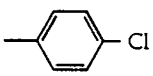 | H | $CH_3$ | H | H | H | together $-O-CH_2-O-$ | |
| Q | 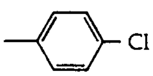 | $-O-\overset{\overset{O}{\|}}{C}-CH_3$ | $CH_3$ | H | H | H | $OCH_3$ | $OCH_3$ |
| R | 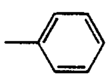 | H | $CH_3$ | H | H | H | $OCH_3$ | $OCH_3$ |
| S | 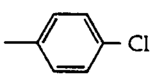 | $-O-\overset{\overset{O}{\|}}{C}-CH_2CH_3$ | $CH_3$ | H | H | H | $OCH_3$ | $OCH_3$ |
| T | 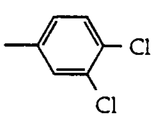 | H | $CH_3$ | H | H | H | OH | OH |
| U | 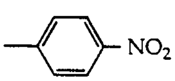 | H | $CH_3$ | H | H | H | $OCH_3$ | $OCH_3$ |
| V | 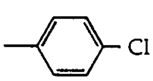 | OH | $CH_3$ | H | H | H | $OCH_3$ | $OCH_3$ |
| W | 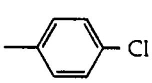 | H | $CH_3$ | H | H | $OCH_3$ | $OCH_3$ | H |

| | | | | | | | | |
|---|---|---|---|---|---|---|---|---|
| X | 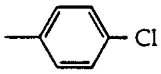 | H | $CH_3$ | $CH_3$ | H | H | $CH_3$ | $CH_3$ |
| AA | 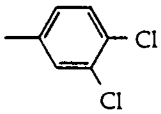 | H | $CH_3$ | H | H | H | $OCH_3$ | $OCH_3$ |

Test results

| Compound | 3H-MK 801 / $IC_{50}$ (μM) | | Electrophysiology/$IC_{50}$ (μM) oocytes | |
|---|---|---|---|---|
| | high | low | NMDAR 1C & 2A | NMDAR 1C & 2B |
| A | 0.04 | 223 | >100 | ≤0.1 |
| B | 0.09 | 54 | 15 | 0.49 |
| C | 0.12 | 191 | >20 | 0.043 |
| D | 0.29 | 116 | 19 | 0.28 |
| E | 0.34 | 129 | | |
| F | 0.4 | 315 | >30 | <0.1 |
| G | 0.46 | 87 | | |
| H | 0.5 | 589 | | |
| I | 0.59 | 146 | | |
| J | 0.6 | 107 | | |
| K | 0.91 | 613 | | |
| L | 1.37 | 198 | | |
| M | 1.39 | 95 | | |
| N | 1.59 | 370 | | |
| O | 1.6 | 101 | | |
| P | 1.7 | 95 | | |
| Q | 1.76 | 161 | 21 | 1.2 |
| R | 2.1 | 147 | | |
| S | 2.18 | 123 | | |
| T | 2.31 | 56 | | |
| U | 2.71 | 87 | | |
| V | 2.9 | 110 | | |
| W | 3.6 | 129 | | |
| X | 3.87 | 2233 | | |
| Y | 3.9 | 93 | | |
| Z | 4.1 | 135 | | |
| AA | 4.62 | 185 | | |

By screening of compounds of formula I this chemical class of compounds could be identified as NMDA receptor subtype selective blockers and - for selected compounds - the preference for NMDAR-2B subunits could be demonstrated by electrophysiological characterization using cloned NMDA receptor subtypes expressed in oocytes.

The compounds of formula I and their salts, as herein described, can be incorporated into standard pharmaceutical dosage forms, for example, for oral or parenteral application with the usual pharmaceutical adjuvant materials, for example, organic or inorganic inert carrier materials, such as, water, gelatin, lactose, starch, magnesium stearate, talc, vegetable oils, gums, polyalkylene-glycols and the like. The pharmaceutical preparations can be employed in a solid form, for example, as tablets, suppositories, capsules, or in liquid form, for example, as solutions, suspensions or emulsions. Pharmaceutical adjuvant materials can be added and include preservatives, stabilizers, wetting or emulsifying agents, salts to change the osmotic pressure or to act as buffers. The pharmaceutical preparations can also contain other therapeutically active substances.

The daily dose of compounds of formula I to be administered varies with the particular compound employed, the chosen route of administration and the recipient. Representative of a method for administering the compounds of formula I is by the oral and parenteral type administration route. An oral formulation of a compound of formula I is preferably administered to an adult at a dose in the range of 150 mg to 1.5 mg per day. A parenteral formulation of a compound of formula I is preferably administered to an adult at a dose in the range of from 5 to 500 mg per day.

The invention is further illustrated in the following examples.

EXAMPLE 1

| Tablet Formulation (Wet Granulation) | | | | |
|---|---|---|---|---|
| Item Ingredients | mg/tablet | | | |
| | 5 mg | 25 mg | 100mg | 500 mg |
| 1. 2-(6,7-Dimethoxy-2-methyl-1,2,3,4-tetrahydroisoquinolin-1-yl)-1-p-tolyl-ethanol | 5 | 25 | 100 | 500 |
| 2. Lactose Anhydrous DTG | 125 | 105 | 30 | 150 |
| 3. Sta-Rx 1500 | 6 | 6 | 6 | 30 |
| 4. Microcrystalline Cellulose | 30 | 30 | 30 | 150 |
| 5. Magnesium Stearate | 1 | 1 | 1 | 1 |
| TOTAL | 167 | 167 | 167 | 835 |

Manufacturing Procedure:

1. Mix Items 1, 2, 3 and 4 and granulate with purified water.
2. Dry the granulation at 50°C.
3. Pass the granulation through suitable milling equipment.
4. Add Item 5 and mix for three minutes; compress on a suitable press.

EXAMPLE 2

| Capsule Formulation | | | | |
|---|---|---|---|---|
| Item Ingredients | mg/capsule | | | |
| | 5 mg | 25 mg | 100mg | 500 mg |
| 1. 2-(6,7-Dimethoxy-2-methyl-1,2,3,4-tetrahydroisoquinolin-1-yl)-1-p-tolyl-ethanol | 5 | 25 | 100 | 500 |
| 2. Hydrous Lactose | 159 | 123 | 148 | --- |
| 3. Corn Starch | 25 | 35 | 40 | 70 |
| 4. Talc | 10 | 15 | 10 | 25 |
| 5. Magnesium Stearate | 1 | 2 | 2 | 5 |
| TOTAL | 200 | 200 | 300 | 600 |

Manufacturing Procedure:

1. Mix Items 1, 2, and 3 in a suitable mixer for 30 minutes.

2. Add Items 4 and 5 and mix for 3 minutes.

3. Fill into a suitable capsule.

EXAMPLE 3

| Tablet Formulation (Wet Granulation) | | | | |
|---|---|---|---|---|
| Item Ingredients | mg/tablet | | | |
| | 5 mg | 25 mg | 100mg | 500 mg |
| 1. 2-(6,7-Dimethoxy-2-methyl-1,2,3,4-tetrahydroisoquinolin-1-yl)-1-p-tolyl-ethanol | 5 | 25 | 100 | 500 |
| 2. Lactose Anhydrous | 125 | 105 | 30 | 150 |
| 3. Sta-Rx 1500 | 6 | 6 | 6 | 30 |
| 4. Microcrystalline Cellulose | 30 | 30 | 30 | 150 |
| 5. Magnesium Stearate | 1 | 2 | 2 | 5 |
| TOTAL | 167 | 167 | 167 | 835 |

Manufacturing Procedure:

1. Mix Items 1, 2, 3 and 4 and granulate with purified water.

2. Dry the granulation at 50°C.

3. Pass the granulation through suitable milling equipment.

4. Add Item 5 and mix for three minutes; compress on a suitable press.

**Claims**

**1.** The use of tetrahydroisoquinoline derivatives of the general formula

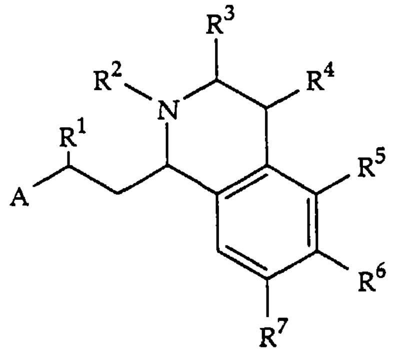

I

wherein

| | |
|---|---|
| A | is aryl |
| $R^1$ | is hydrogen, hydroxy, lower alkyl, lower alkoxy, R-CO- or R-COO-, wherein R is lower alkyl; |
| $R^2$ | is hydrogen, lower alkyl or cycloalkyl |
| $R^3$-$R^7$ | are hydrogen, lower alkyl, lower alkoxy, hydroxy or |
| $R^3$ and $R^4$ | taken together are $-(CH_2)_n-$ or |
| $R^6$ and $R^7$ | taken together are $-OCH_2O-$ and |
| n | is 3 or 4, |

as well as pharmaceutically acceptable salts for the manufacture of medicaments for the control or treatment of diseases which represent therapeutic indications for NMDA receptor subtype specific blockers.

**2.** The use of compounds of formula I according to claim 1, wherein the therapeutic indications include acute forms of neurodegeneration caused e.g. by stroke and brain trauma, and chronic forms of neurodegeneration such as Alzheimer's disease, Parkinson's disease, Huntington's disease, ALS (amyotrophic laterial slcerosis) and neurodegeneration associated with bacterial or viral infections, and, in addition, therapeutic indications such as schizophrenia, anxiety and depression.

**3.** The use of

2-(6,7-Dimethoxy-2-methyl-1,2,3,4-tetrahydroisoquinolin-1-yl)-1-p-tolyl-ethanol;
1-[2-(4-Chloro-phenyl)-ethyl]-6-methoxy-2-methyl-1,2,3,4-tetrahydroisoquinolin-7-ol;
1-(4-Chloro-phenyl)-2-(6,7-dimethoxy-2-methyl-1,2,3,4-tetrahydroisoquinolin-1-yl)-ethanol;
1-[2-(4-Chloro-phenyl)-ethyl]-2-methyl-1,2,3,4-tetrahydro-isoquinline-6,7-diol;
6,7-Dimethoxy-2-methyl-1-(2-p-tolyl-ethyl)-1,2,3,4-tetrahydroisoquinline;
6-[2-(4-Chloro-phenyl)-ethyl]-8,9-dimethoxy-5-methyl-1,2,3,4,4a,5,6,10b-octahydro-phenanthridine;
2-(6,7-Dimethoxy-2-methyl-1,2,3,4-tetrahydroisoquinlin-1-yl)-1-(4-nitrophenyl)-ethanol; or
6-[2-(4-Chloro-phenyl)-ethyl]-8,9-dimethoxy-1,2,3,4,4a,5,6,10b-octahydrophenanthridine

according to claim 1 or 2.

**4.** Compounds of formula

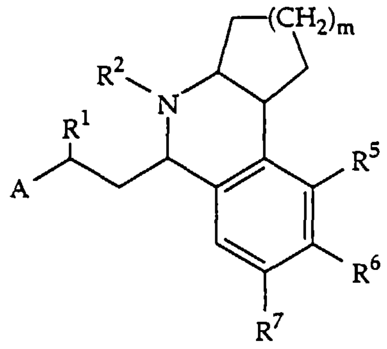

Ia

wherein A, $R^1$, $R^2$ and $R^5$-$R^7$ are as in claim 1 and m is 1 or 2.

**5.** A medicament containing one or more compounds of formula I according to claim 1 for the treatment of diseases caused by acute forms of neurodegeneration caused e.g. by stroke and brain trauma, and chronic forms of neurodegeneration such as Alzheimer's disease, Parkinson's disease, Huntington's disease, ALS (amyotrophic laterial slcerosis) and neurodegeneration associated with bacterial or viral infections, and, in addition, therapeutic indications such as schizophrenia, anxiety and depression.

European Patent Office

# EUROPEAN SEARCH REPORT

Application Number
EP 97 10 0926

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | US 3 823 148 A (A.B.A JANSEN ET AL)<br>* column 1, lines 14-28; column 2, lines 38-44; example 24 * | 1,2,5 | A61K31/47<br>C07D221/12<br>C07D221/16<br>C07D217/20<br>C07D217/18<br>C07D217/04<br>C07D491/04 |
| A | CHEM. PHARM. BULL.,<br>vol. 44, no. 1, January 1996,<br>pages 95-102, XP000674300<br>M. OHKUBO ET AL:<br>* page 96 - page 99 * | 1,2,5 | |
| A,D | US 3 238 212 A (A. BROSSI ET AL)<br>* column 3, lines 44-48; examples 1-3; column 6, lines 29-31 * | 1-3,5 | |
| A,D | US 3 217 007 A (A. BROSSI ET AL)<br>* column 3, line 67-75; example 2 * | 1-3,5 | |
| A | HELV. CHIM. ACTA,<br>vol. 43, 1960,<br>pages 1459-1472, XP000674281<br>A. BROSSI ET AL:<br>* pages 1461 and 1467, compound XVII; table 5, entry 6; pages 1463-1464 * | 1-3,5 | TECHNICAL FIELDS SEARCHED (Int.Cl.6)<br>A61K<br>C07D |
| A,D | US 3 067 203 A (H. BESENDORF ET AL)<br>* column 3, lines 8-14; examples, claims 1-7, 9 * | 1,2,5 | |
| A | CHEMICAL ABSTRACTS, vol. 77, no. 1,<br>3 July 1972<br>Columbus, Ohio, US;<br>abstract no. 5364,<br>XP002032420<br>* abstract; RN 33543-58-7 *<br>& JP 47 007 381 B (GRELAN PHARMACEUTICAL CO LTD) | 1,2,5 | |

-/--

The present search report has been drawn up for all claims

| Place of search | Date of completion of the search | Examiner |
|---|---|---|
| BERLIN | 5 June 1997 | Van Amsterdam, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 97 10 0926

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | GB 976 829 A (F. HOFFMANN-LA ROCHE & CO AG)<br>* page 1, line 9 - line 49 *<br>----- | 1,4 | |

TECHNICAL FIELDS SEARCHED (Int.Cl.6)

The present search report has been drawn up for all claims

| Place of search | Date of completion of the search | Examiner |
|---|---|---|
| BERLIN | 5 June 1997 | Van Amsterdam, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)